# EUROPÄISCHE PATENTANMELDUNG

(11) **EP 4 678 123 A1**
(43) Veröffentlichungstag der Anmeldung: **14.01.2026**
(21) Anmeldenummer: 25185472.5
(22) Anmeldetag: 26.06.2025
(51) Int. Cl.: A61B 17/29, A61B 34/30

(54) **MEDIZINISCHES INSTRUMENT**

(30) Priorität: 10.07.2024 DE 102024119580
(71) Anmelder: Karl Storz SE & Co. KG, 78532 Tuttlingen (DE)
(72) Erfinder: Hoffmann, Martin, 13187 Berlin (DE)

(57) **Zusammenfassung**

Die Erfindung betrifft ein Medizinisches Instrument (10) umfassend:
- zwei zumindest entlang ihrer Längsrichtung bewegbare Antriebselemente (12) und
- einen Endeffektor (14) mit wenigstens zwei zusammenwirkenden Endeffektorelementen (16),
wobei die Endeffektorelemente (16) jeweils an eines der Antriebselemente (12) bewegungsübertragend gekoppelt und unabhängig voneinander um eine Schwenkachse (S) verschwenkbar sind.

## Beschreibung

Die vorliegende Erfindung betrifft ein medizinisches Instrument.

Aufgrund ihrer zuverlässigen und präzisen Steuerung ersetzen medizinische Robotersysteme und/oder zumindest teilweise elektrisch betriebene medizinische Instrumente zunehmend konventionelle, manuell betreibbare medizinische Instrumente. Um die benötigte Flexibilität und/oder Gelenkigkeit der medizinischen Instrumente bereitstellen zu können, weisen derzeit bekannte medizinische Instrumente einen äußerst komplexen und/oder kostenintensiven Aufbau auf. Ferner weisen in Robotersystemen eingesetzte herkömmliche medizinische Instrumente, thermisch instabile Komponenten, beispielsweise in Form von Seilanordnungen auf, sodass eine Wiederverwendung derartiger medizinischer Instrumente, insbesondere nach einem oder mehreren Sterilisationsprozessen, nur bedingt möglich ist. Da somit regelmäßige Neuanschaffungen erforderlich sind, werden nicht nur die ökologische, sondern auch die ökonomische Nachhaltigkeit solcher medizinischen Instrumente maßgeblich beeinträchtigt,

Der Erfindung liegt insbesondere aber nicht beschränkt darauf die Aufgabe zugrunde, ein medizinisches Instrument, insbesondere für den Einsatz in einem medizinischen Robotersystem, vorteilhaft weiterzuentwickeln, vor allem hinsichtlich einer Erhöhung einer Bewegungsfreiheit sowie einer Zuverlässigkeit. Ferner ist es unter anderem eine Aufgabe der vorliegenden Erfindung eine Wiederverwendbarkeit eines solchen medizinischen Instruments zu gewährleisten und dadurch Betriebskosten zu senken.

Diese Aufgabe wird erfindungsgemäß durch die Merkmale der unabhängigen Ansprüche gelöst. Weiterbildungen der Erfindung sind den abhängigen Ansprüchen zu entnehmen.

Die Erfindung betrifft ein medizinisches Instrument umfassend:
- zwei zumindest entlang ihrer Längsrichtung bewegbare Antriebselemente und
- einen Endeffektor mit wenigstens zwei zusammenwirkenden Endeffektorelementen, wobei die Endeffektorelemente jeweils an eines der Antriebselemente bewegungsübertragend gekoppelt und unabhängig voneinander um eine Schwenkachse verschwenkbar sind.

Durch eine derartige Ausgestaltung kann ein vorteilhaft weiterentwickeltes medizinisches Instrument bereitgestellt werden. Insbesondere kann ein solches medizinisches Instrument eine hohe Bewegungsfreiheit und Zuverlässigkeit aufweisen. Zudem kann ein Kostenaufwand für medizinische Einsätze reduziert werden, da ein derartiges medizinisches Instrument wiederverwendet werden kann.

Unter einem "medizinischen Instrument" soll insbesondere ein medizinisches Werkzeug verstanden werden, das vorzugsweise dazu eingerichtet ist, ein zu bearbeitendes Objekt zu greifen, zu manipulieren, zu halten, zu schneiden und/oder anderweitig mit dem zu bearbeitenden Objekt zu interagieren.

Unter "eingerichtet" soll speziell programmiert, vorgesehen, ausgelegt, ausgebildet und/oder ausgestattet verstanden werden. Darunter, dass ein Objekt zu einer bestimmten Funktion eingerichtet ist, soll ferner verstanden werden, dass das Objekt diese bestimmte Funktion in zumindest einem Anwendungs- oder Betriebszustand erfüllt oder ausführt.

Das medizinische Instrument kann für den Einsatz in chirurgischen Eingriffen und/oder invasiven Operationen vorgesehen sein. Das medizinische Instrument kann Teil eines medizinischen Robotersystems und/oder zumindest mit einem solchen funktionsfähig koppelbar sein. In einigen Ausführungsformen kann das medizinische Instrument als ein handhaltbares medizinisches Instrument ausgestaltet sein.

Ein zu bearbeitendes Objekt kann sich auf jegliche organische und/oder anorganische Struktur beziehen. Insbesondere sind damit anatomische Strukturen eines Patienten, wie beispielsweise Organe und/oder Gewebe gemeint und/oder Verbrauchsmaterial, wie beispielsweise, Fäden, Heftklammern, Folien, Tupfer, Schläuche, Schrauben und/oder Nägel gemeint.

Bei einem Antriebselement soll es sich vorzugsweise um eine Komponente und/oder Vorrichtung handeln, die dazu eingerichtet ist, Bewegungen zu erzeugen, zu übertragen und/oder zu steuern.

Unter einem "Endeffektor" soll insbesondere eine Komponente und/oder Vorrichtung des medizinischen Instruments verstanden werden, die vorzugsweise am distalen Ende des medizinischen Instruments, oder in anderen Worten, während eines Einsatzes des medizinischen Instruments, patientennah, angeordnet ist. Der Endeffektor ist dazu eingerichtet, physischen Kontakt mit einem und/oder mehreren zu bearbeitenden Objekten herzustellen und/oder mit diesen zu interagieren. Der Endeffektor kann je nach Anforderungsbereich und/oder Aufgabe unterschiedlich ausgebildet sein.

Unter "wenigstens zwei zusammenwirkenden Effektorelementen" sollen wenigstens zwei Teilkomponenten des Endeffektors verstanden werden, die miteinander interagieren, kooperieren und/oder sich gegenseitig beeinflussen, um eine bestimmte Aufgabe auszuführen und/oder zu erfüllen. Die beiden Effektorelemente können komplementär ausgebildet sein. Die Effektorelemente können einen Formschluss bilden.

Dass die Endeffektorelemente unabhängig voneinander um wenigstens eine Schwenkachse verschwenkbar sind, kann bedeuten, dass die beiden Endeffektorelemente separat und/oder ohne direkte Beeinflussung durch eine Bewegung des anderen Effektorelements verschwenkt werden können. Die Schwenkachse kann senkrecht zu der Längsrichtung der Antriebselemente verlaufen. Bei der Schwenkachse kann es sich um eine Bezugsachse handeln, die sich auf einen einzelnen Freiheitsgrad beziehen kann, der eine Schwenkbewegung der Endeffektorelemente um eine jene Schwenkachse ermöglichen kann. Die Schwenkbewegung der Endeffektorelemente kann in vordefinierten Winkelschritten erfolgen, ist bevorzugt jedoch nahezu stufenlos und besonders bevorzugt stufenlos. Die Endeffektorelemente können jeweils um zumindest 45°, bevorzugt zumindest 90° und besonders bevorzugt zumindest 160° um die Schwenkachse verschwenkbar sein. Die Endeffektorelemente können als Maulteile ausgebildet sein.

Gemäß einer Weiterbildung kann das medizinische Instrument wenigstens zwei Abtriebselemente umfassen, die jeweils drehfest mit einem der Endeffektorelemente verbunden sind. Die Abtriebselemente können jeweils mit einem der Antriebselemente bewegungsübertragend und/oder bewegungsübertragbar gekoppelt sein. Die Abtriebselemente können dazu eingerichtet sein, Kräfte und/oder Bewegungen der Antriebselemente aufzunehmen. Dadurch kann eine zuverlässige und/oder präzise Bewegungsübertragung zwischen den Antriebselementen und den Abtriebselementen und/oder den Endeffektorelementen gewährleistet werden.

In einigen Ausführungsformen kann wenigstens eines der Abtriebselemente als ein Zahnrad ausgebildet sein. Wenigstens eines der Antriebselemente kann als eine mit einem der Abtriebselemente und/oder mit dem Zahnrad kooperierende Zahnstange ausgebildet sein. In einigen Ausführungsformen können die Abtriebselemente und die Endeffektorelemente einteilig und/oder monolithisch ausgebildet sein. Die Zahnräder und oder Zahnstangen können vorzugsweise aus thermisch stabilen Materialien, wie Metallen und/oder Hochleistungspolymeren, wie beispielsweise Polyetherketonen, gefertigt sein. In einigen Ausführungsformen können die Antriebs- und/oder Abtriebselemente auch andere dem Fachmann als vorteilhaft erscheinende mechanische Komponenten und/oder Antriebsmechanismen umfassen, wie beispielsweise Schneckengetriebe und/oder Malterserkreuzgetriebe. Mittels einer derartigen Ausgestaltung können Bewegungen durch Abrollbewegungen zuverlässig und/oder präzise übertragen werden. Eine derartige Ausgestaltung kann robust gegenüber Verschleiß und/oder hohen Temperaturen sein, wodurch die Lebensdauer des medizinischen Instruments, trotz Sterilisationsverfahren bei hohen Temperaturbereichen und/oder häufiger Nutzung, signifikant erhöht wird.

Gemäß einer Weiterbildung kann das medizinische Instrument einen Schaft mit einem distalen Schaftabschnitt umfassen, in welchem die Antriebselemente zumindest abschnittsweise angeordnet sind. Bei dem Schaft kann es sich um ein längliches und/oder zylindrisches Bauteil handeln. Durch den Schaft können mechanische Komponenten und/oder Leitungen sicher und/oder geordnet geführt werden. Der Schafft kann zur Stabilität des medizinischen Instruments beitragen und/oder kinematischen Strukturen vor äußeren Einflüssen schützen.

In einigen Ausführungsformen kann das medizinische Instrument wenigstens zwei Aktuatorelemente umfassen. Jeweils eines der Aktuatorelemente kann mit einem der Antriebselemente bewegungsübertragend und/oder bewegungsübertragbar gekoppelt sein. Die Aktuatorelemente können zumindest abschnittsweise in einem proximalen Schaftabschnitt des Schafts angeordnet sein. Ein proximaler Schaftabschnitt kann sich dabei auf einen Abschnitt des Schafts des medizinischen Instruments beziehen, der während eines Betriebs von dem Patienten abgewandt ist. Die Aktuatorelemente können jeweils als eine Zug- und/oder Druckstange ausgebildet sein, die dazu eingerichtet sind, Zug- und/oder Druckkräfte auf ein jeweiliges Antriebselement zu übertragen. In anderen Worten wäre denkbar, dass jedes Aktuatorelement in zwei entgegengesetzte Richtungen entlang einer Längsachse des Schafts bewegt werden kann. Derartige Zug- und/oder Druckstangen bieten eine erhöhte Stabilität, eine effiziente Kraftübertragung und/oder eine bessere Lastverteilung, was zu einer verbesserten Leistung, Zuverlässigkeit und/oder Lebensdauer des medizinischen Instruments beitragen kann.

Gemäß einer Weiterbildung kann das medizinische Instrument wenigstens zwei Kopplungselemente umfassen, welche jeweils eines der Antriebselemente mit einem der Aktuatorelemente gelenkig, verliersicher, bewegungsübertragend und/oder bewegungsübertragbar koppeln. Die Kopplungselemente können zumindest abschnittsweise parallel angeordnet sein. Auf diese Weise können der Endeffektor und/oder die Endeffektorelemente effizient und/oder zuverlässig über eine Distanz gesteuert und/oder miteinander gekoppelt werden.

Zudem kann der Schaft des medizinischen Instruments ein Scharniergelenk umfassen, mittels welchem der distale Schaftabschnitt um eine Gelenkachse relativ zum proximalen Schaftabschnitt abknickbar ist. Die Kopplungselemente können in einem Bereich des Scharniergelenks angeordnet sein. Durch diese Ausgestaltung erhält das medizinische Instrument einen weiteren Freiheitsgrad. Der weitere Freiheitsgrad gestattet es dem distalen Schaftabschnitt sich um die Gelenkachse relativ zum proximalen Schaftabschnitt verschwenken zu können. Der distale Schaftabschnitt kann um zumindest 45°, bevorzugt zumindest 90° und besonders bevorzugt zumindest 160° um die Gelenkachse verschwenkbar und/oder abknickbar sein. Diese Gelenkachse kann senkrecht zu der Schwenkachse verlaufen, um welche die Endeffektorelemente verschwenkbar sind. Aufgrund des weiteren Freiheitsgrads kann die Bewegungsfreiheit des medizinischen Instruments und/oder die Präzision verbessern. Dies kann außerdem zu einer besseren Manövrierfähigkeit, einer erweiterten Reichweite und/oder einer effizienteren Durchführung von chirurgischen Eingriffen, insbesondere in schwer zugänglichen Bereichen, wie Kavitäten des Patienten, beitragen. Die Ausrichtung und/oder Steuerung des Scharniergelenks und/oder des distalen Schaftabschnitts kann unabhängig von der Steuerung und/oder Ausrichtung des Endeffektors und/oder der Endeffektorelemente erfolgen.

In einigen Ausführungsformen kann ein Abstand zwischen der Schwenkachse und der Gelenkachse, während einer Benutzung des medizinischen Instruments, konstant sein, wodurch zufällige und/oder parasitäre Bewegungen der Schaftabschnitte und/oder der Endeffektorelemente reduziert und/oder verhindert werden können.

Die erfindungsgemäßen Vorrichtungen sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine perspektivische Ansicht eines medizinischen Instruments mit einer kinematischen Struktur,
- Fig. 2: eine Detailansicht der kinematischen Struktur des medizinischen Instruments in einer ersten Position,
- Fig. 3: eine Detailansicht der kinematischen Struktur des medizinischen Instruments in der ersten Position,
- Fig. 4: eine Detailansicht einer Hälfte der kinematischen Struktur des medizinischen Instruments,
- Fig. 5: eine Detailansicht der kinematischen Struktur des medizinischen Instruments in einer zweiten Position,
- Fig. 6: eine Seitenansicht der kinematischen Struktur des medizinischen Instruments gemäß Fig. 5,
- Fig. 7: eine Seitenansicht der kinematischen Struktur des medizinischen Instruments in einer dritten Position,
- Fig. 8: eine Detailansicht eines Endeffektors in einer ersten Position,
- Fig. 9: eine Detailansicht eines Endeffektors in einer zweiten Position, und
- Fig.10: eine Seitenansicht einer eines medizinischen Instruments gemäß einer weiteren Ausführungsform.

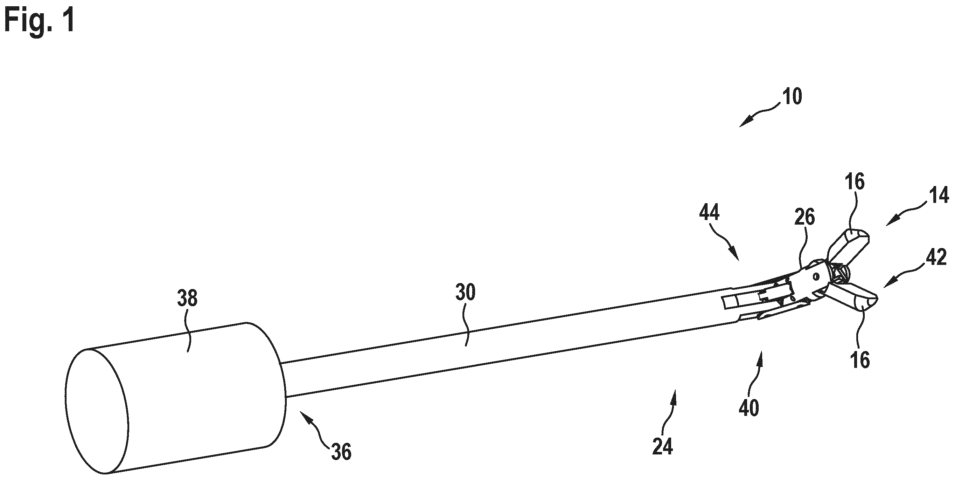

In Fig. 1 ist ein medizinisches Instrument 10 mit einem Schaft 24 mit einem proximalen Schaftabschnitt 30 und einem distalen Schaftabschnitt 26 gezeigt. An einem proximalem Ende 36 des proximalen Schaftabschnitts 30 ist eine Schnittstelle 38 des medizinischen Instruments 10 angeordnet. Die Schnittstelle 38 kann dazu eingerichtet sein, mit einer hierin nicht gezeigten Robotervorrichtung und/oder einer Handhabe funktionsfähig und/oder steuerbar gekoppelt zu werden. An einem distalem Ende 40 des proximalen Schaftabschnitts 30 ist der distaler Schaftabschnitt 26 des medizinischen Instruments 10 mit einem Endeffektor 14 angeordnet. Der Endeffektor 14 umfasst eine Greifanordnung 42 mit zwei Endeffektorelementen 16.

An dieser Stelle sei darauf hingewiesen, dass die Greifanordnung 42 lediglich beispielhaft zu verstehen ist und je nach Anwendungsbereich auch andere dem Fachmann vorteilhaft erscheinende Endeffektoren und/oder Anordnungen vorgesehen seien können. Eine Länge des proximalen Schaftabschnitts 30 kann insbesondere zumindest dem fünffachen, vorzugsweise zumindest dem zehnfachen und besonders bevorzugt zumindest dem fünfzehnfachen der Länge des distalen Schaftabschnitts 26 entsprechen.

Der Endeffektor 14, die Endeffektorelemente 16 und/oder der distale Schaftabschnitt 26 lassen sich mittels einer kinematischen Struktur 44 des medizinischen Instruments 10 steuern und/oder bewegen.

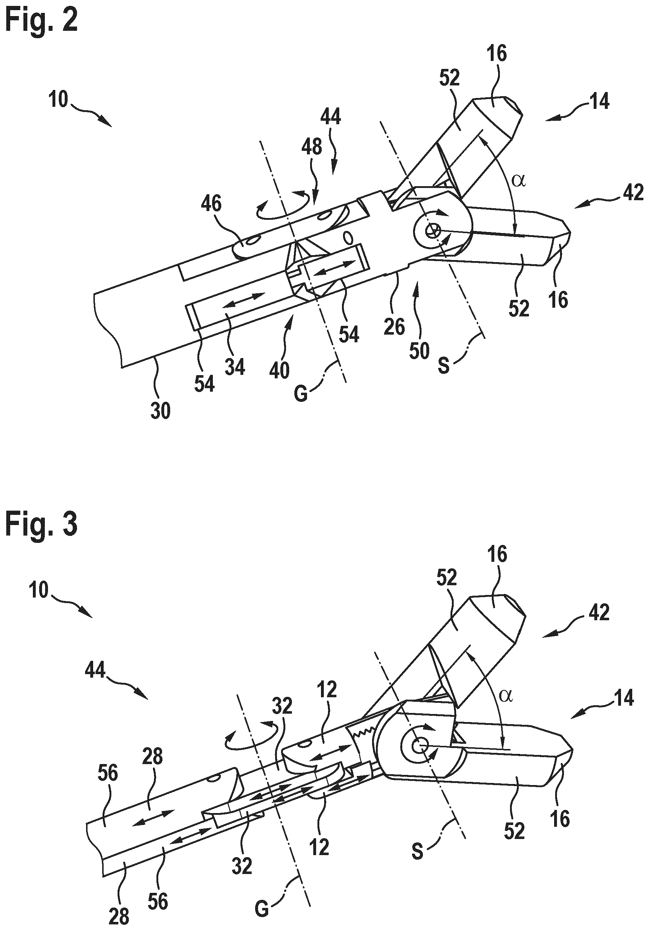

In Fig. 2 ist eine Detailansicht der kinematischen Struktur 44 in einer ersten Position gezeigt. Zu erkennen ist das distale Schaftende 40 des proximalen Schaftabschnitts 30, welches mittels zweier Laschenelemente 46 verliersicher und beweglich mit einem proximalen Schaftende 48 des distalen Schaftabschnitt 26 gekoppelt ist. Von den Laschenelementen 46 ist lediglich eines zu sehen, das andere Laschenelement 46 ist in dieser Ansicht verdeckt. Die Laschenelemente 46 beabstanden den distalen Schaftabschnitt 26 von dem proximalen Schaftabschnitt 30 und gewährleisten einen konstanten Abstand zwischen dem distalen Schaftende 40 des proximalen Schaftabschnitts 30 und dem proximalen Ende 48 des distalen Schaftabschnitts 36.

Ferner umfasst das medizinische Instrument 10 und/oder die kinematische Struktur 44 ein Scharniergelenk 34, mittels welchem der distale Schaftabschnitt 26 und eine Gelenkachse G relativ zum proximalen Schaftabschnitt 30 abknickbar ist. Das Scharniergelenk 34 wird jeweils in und/oder entlang einer an dem proximalen Schaftabschnitt 30 und dem distalen Schaftabschnitt 26 ausgebildeten Führungsnut 54 geführt. Eine Schwenkachse S, um welche die Endeffektorelemente 16 jeweils unabhängig voneinander verschwenkbar sind sowie die Gelenkachse G, sind durch gestrichelte Linien angedeutet. Durch die Gelenkachse G und/oder die Schwenkachse S erlangte Freiheitsgrade der Endeffektorekemente 16 und/oder des distalen Schaftabschnitts 26, sind als Pfeile angedeutet.

Der Endeffektor 14 ist an einem distalen Schaftende 50 des distalen Schaftabschnitts 26 angeordnet und weist zwei Endeffektorelementen 16 auf. Die beiden Endeffektorelemente 16 sind in der hierin gezeigten Ausführungsform jeweils als ein Maulteil 50 konfiguriert. Die beiden Maulteile 52 sind komplementär ausgebildet, sodass sie mit dem jeweils anderen Maulteil 52 zusammenwirken können. Die beiden Maulteile 52 sind unabhängig voneinander um die Schwenkachse S verschwenkbar. Die beiden Endeffektorelemente 16 sind in der hierin gezeigten Position in einem ersten Winkel α relativ zueinander um die Schwenkachse S verschwenkt. Die Schwenkachse S und die Gelenkachse G verlaufen senkrecht zueinander.

Die in der Fig. 3 dargestellten Detailansicht der kinematischen Struktur 44 unterscheidet sich von der in Fig. 2 dargestellten insofern, dass in Fig. 3 der proximale Schaftabschnitt 30, der distale Schaftabschnitt 26, sowie das Scharniergelenk 34 ausgeblendet sind. Ohne die Schaftabschnitte 30, 26 sind weitere Elemente der kinematischen Struktur 44 zu erkennen. Zu erkennen sind zwei Antriebselemente 12 der kinematischen Struktur 44, die bewegungsübertragend und/oder bewegungsübertragbar mit an den Endeffektorelementen angeordneten Abtriebselementen 18 gekoppelt sind. Die Abtriebselemente 18 und/oder die Antriebselemente 12 sind in einem Bereich des distalen Schaftabschnitts 26 angeordnet.

In der vorliegend gezeigten Ausführungsform sind die Antriebselemente 12 als Zahnstangen 22 ausgebildet. Ferner zu erkennen sind zwei Aktuatorelemente 28 der kinematischen Struktur 44, die hierin als Zug- und/oder Druckstangen 56 ausgebildet sind. Die beiden Aktuatorelemente 28 sind in einem Bereich des proximalen Schaftabschnitts 26 angeordnet und verlaufen parallel zueinander. Jedes der beiden Aktuatorelemente 28 kann sich unabhängig vom jeweils anderen Aktuatorelement 28 in einer Linearbewegung bewegen. Freiheitsgrade der Aktuatorelemente 28 sind entsprechend durch Pfeile angedeutet.

Jedes der beiden Aktuatorelemente 28 ist über jeweils ein Kopplungselement 32 bewegungsübertragend und/oder bewegungsübertragbar mit einem der Antriebselemente 12 gelenkig gekoppelt.

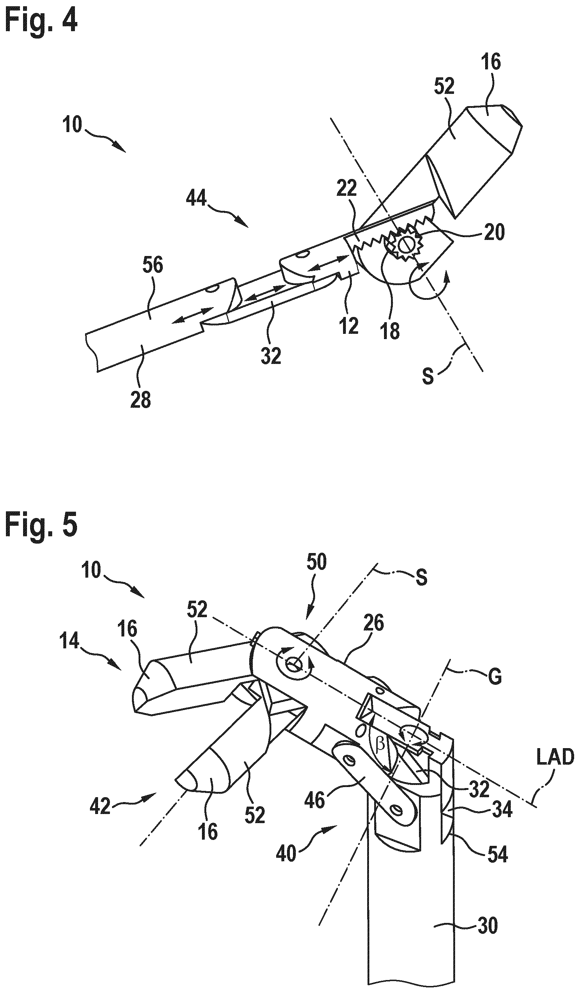

In Fig. 4 ist eine Detailansicht einer Hälfte der kinematischen Struktur 44 des medizinischen Instruments 10 gezeigt. In dieser Figur ist im Vergleich zu Fig. 3 lediglich ein Aktuatorelement 28, ein Kopplungselement 32, ein Antriebselement 12 sowie ein Endeffektorelement 16 dargestellt. Des Weiteren ist in Fig. 4 das Abtriebselement 18 zu erkennen, welches hierin in Form eines Zahnrads 20 ausgebildet ist und mit der Zahnstange 22 bewegungsübertragend und/oder bewegungsübertragbar gekoppelt ist. Pfeile sollen schematisch die Freiheitsgrade der einzelnen Komponenten andeuten. Eine Linearbewegung des Aktuatorelements 28 bewegt das Kopplungselement 32 sowie das Antriebselement 12 in dieselbe Richtung, wodurch die Linearbewegung des Antriebselements 12 letztendlich an der Stelle, an der das Antriebselement 18 mit dem Abtriebselement 18 gekoppelt ist, in eine Rotationsbewegung des Abtriebselements 18 transformiert wird.

Fig. 5 stellt eine Detailansicht der kinematischen Struktur 44 des medizinischen Instruments 10 in einer zweiten Position dar. Der Unterschied zu der in den in den Figuren 1 bis 4 gezeigten Position und/oder dem Zustand der kinematischen Struktur 44, besteht in der Orientierung des proximalen Schaftabschnitts 30 relativ zum distalen Schaftabschnitt 26. In dem gezeigten zweiten Zustand wurde das Scharniergelenk 34 in Richtung des distalen Schaftabschnitts 26 geschoben, sodass der distale Schaftabschnitt 26 in einem zweiten Winkel β um die Gelenkachse G relativ zum proximalen Schaftabschnitt 30 abgeknickt ist. Die Führungsnuten 54, in welchen das Scharniergelenk 34 geführt wird, ist sowohl auf der Seite des proximalen Schaftabschnitts 30 als auch auf der Seite des distalen Schaftabschnitts 26 begrenzt. Diese Begrenzung kann als eine Art Anschlagelement fungieren, das eine Bewegung des Scharniergelenks 34 in die eine und/oder die andere Richtung zulässt und/oder begrenzt.

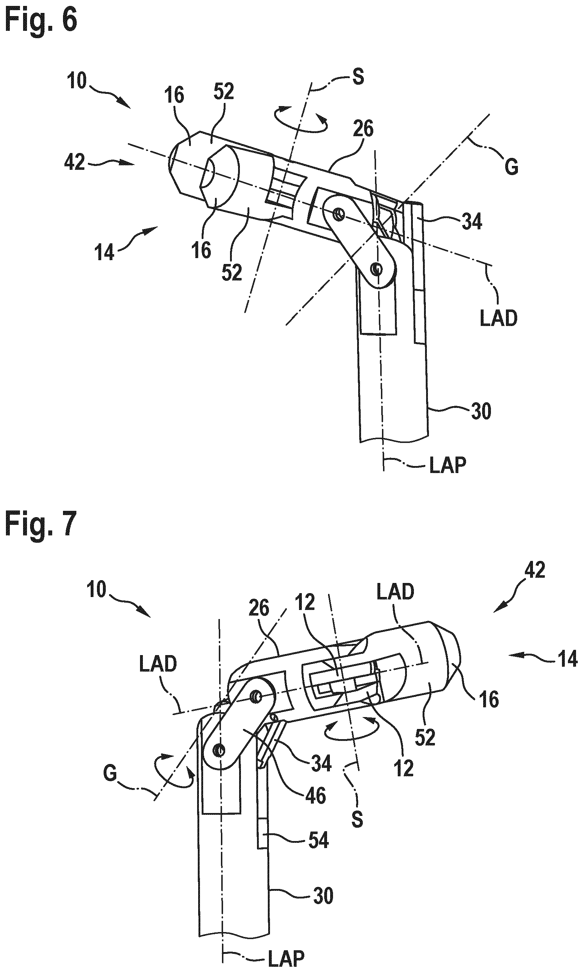

Die Figuren 6 und 7 zeigen eine Verschwenkung des distalen Schaftabschnitts 26 um die Gelenkachse G. Die Position und/oder der Zustand, in der/dem sich die kinematische Struktur 44 in Fig. 6 befindet, gleicht der in Figur 5 gezeigten Position. Durch eine Bewegung des Scharniergelenks 34 in Richtung des distalen Schaftabschnitts 26, werden der distale Schaftabschnitt 26 und das Scharniergelenk 34 und/oder die Längsachse des distalen Schaftabschnitts LAD um einen Winkelbetrag relativ zur Längsachse LAP des proximalen Schaftabschnitts abgeknickt. Die Endeffektorelemente sind durch Stellung der Aktuatorelemente 28 und/oder der Antriebselemente 12, um einen Winkelbetrag relativ zur Längsachse des distalen Schaftabschnitts 26 verschwenkt.

In Figur 7 wurde das Scharniergelenk 34 in die entgegengesetzte Richtung, also in Richtung des proximalen Schaftendes 36 des proximalen Schaftabschnitts 30 bewegt, sodass der distale Schaftabschnitt in die entgegengesetzte Richtung um etwa 160° verschwenkt wurde.

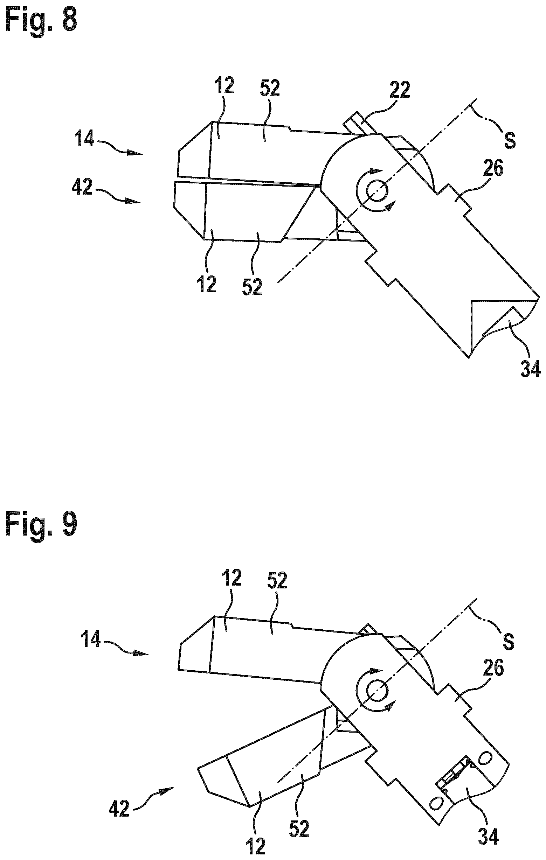

Die Figuren 8 und 9 zeigen eine Detailansicht eines Endeffektors und/ oder der Endeffektorelemente in unterschiedlichen Positionen und/oder Zuständen. Gemäß Figur 8 sind die beiden als Maulteile 52 ausgebildeten Endeffektorelemente 16 in einer geschlossenen und/oder Greifposition ausgereichtet. Die Endeffektorelemente 16 sind im Wesentlichen formschlüssig ausgebildet, wodurch eine hohe Klemmkraft gewährleistet wird. Durch Steuerung und/oder Bewegungen der Aktuatorelemente 28 und daraus resultierenden Bewegungen der Antriebselemente 12 können die Endeffektorelemente 16 unabhängig voneinander und/oder relativ zueinander, in eine in Figur 9 gezeigte offene und/oder Freigabeposition verschwenkt werden.

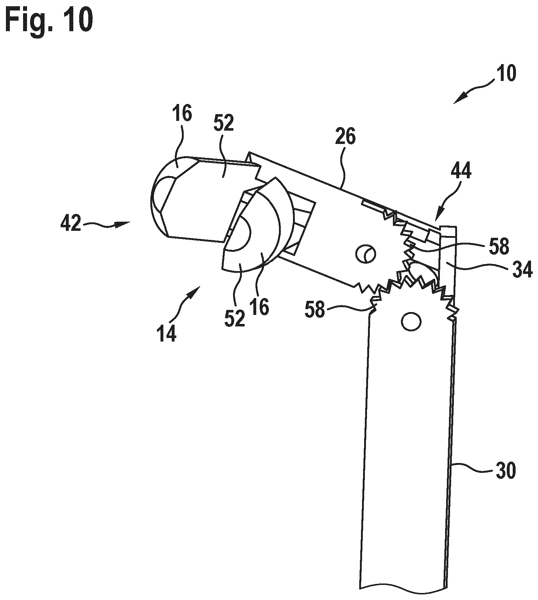

In Figur 10 ist eine weitere Ausführungsform eines medizinischen Instruments gezeigt. Gemäß dieser Ausführungsform sind die Schaftenden 40, 48 des proximalen und distalen Schafabschnitts 26, 30 als aufeinander abrollende Zahnradprofile 58 ausgebildet. Die Ausgestaltung als aufeinander abrollende Zahnradprofile 58 stellt eine besonders einfache konstruktive Lösung dar, die es aufgrund des zwischen den beiderseitigen Zähnen auftretenden Form- und Reibschlusses ermöglicht, den distalen Schaftabschnitt 26 in die unterschiedlichsten abgewinkelten Positionen sicher und/oder zuverlässig bewegen und/oder in gewünschten Positionen zu halten. Die Laschenelemente 46 sind der Übersichtlichkeit halber in der Fig. 10 ausgeblendet.

### Bezugszeichenliste

- 10: Medizinisches Instrument
- 12: Antriebselement
- 14: Endeffektor
- 16: Endeffektorelement
- 18: Abtriebselement
- 20: Zahnrad
- 22: Zahnstange
- 24: Schaft
- 26: distaler Schaftabschnitt
- 28: Aktuatorelement
- 30: proximaler Schaftabschnitt
- 32: Kopplungselement
- 34: Scharniergelenk
- 36: proximales Schaftende
- 38: Schnittstelle
- 40: distales Schaftende
- 42: Greifanordnung
- 44: kinematische Struktur
- 46: Laschenelement
- 48: proximales Schaftende
- 50: distales Schaftende
- 52: Maulteil
- 54: Führungsnut
- 56: Zug- und/oder Druckstange
- 58: Zahnradprofil
- S: Schwenkachse
- G: Gelenkachse
- a: erster Winkel
- β: zweiter Winkel
- LAP: Längsachse des proximalen Schaftabschnitts
- LAD: Längsachse des distalen Schaftabschnitts

## Patentansprüche

1. Medizinisches Instrument (10) umfassend:
- zwei zumindest entlang ihrer Längsrichtung bewegbare Antriebselemente (12) und
- einen Endeffektor (14) mit wenigstens zwei zusammenwirkenden Endeffektorelementen (16),
wobei die Endeffektorelemente (16) jeweils an eines der Antriebselemente (12) bewegungsübertragend gekoppelt und unabhängig voneinander um eine Schwenkachse (S) verschwenkbar sind.

2. Medizinisches Instrument (10) nach Anspruch 1, ferner umfassend:
- wenigstens zwei Abtriebselemente (18), die jeweils drehfest mit einem der Endeffektorelemente (16) verbunden sind,
wobei die Abtriebselemente (18) jeweils mit einem der Antriebselemente (12) bewegungsübertragend gekoppelt sind.

3. Medizinisches Instrument (10) nach Anspruch 2,
wobei wenigstens eines der Abtriebselemente (18) als ein Zahnrad (20) und wenigstens eines der Antriebselemente (12) als eine kooperierende Zahnstange (22) ausgebildet ist.

4. Medizinisches Instrument (10) nach einem der vorherigen Ansprüche, ferner umfassend:
- einen Schaft (24) mit einem distalen Schaftabschnitt (26), in welchem die Antriebselemente (12) zumindest abschnittsweise angeordnet sind.

5. Medizinisches Instrument (10) nach Anspruch 4, ferner umfassend:
- wenigstens zwei Aktuatorelemente (28),
wobei jeweils eines der Aktuatorelemente (28) mit einem der Antriebselemente (12) bewegungsübertragend gekoppelt ist,
und wobei die Aktuatorelemente (28) zumindest abschnittsweise in einem proximalen Schaftabschnitt (30) des Schafts (24) angeordnet sind.

6. Medizinisches Instrument (10) nach Anspruch 5, ferner umfassend:
- wenigstens zwei Kopplungselemente (32), welche jeweils eines der Antriebselemente (12) mit einem der Aktuatorelemente (28) gelenkig und bewegungsübertragend koppeln.

7. Medizinisches Instrument (10) nach Anspruch 6,
wobei der Schaft (24) ein Scharniergelenk (34) umfasst, mittels welchem der distale Schaftabschnitt (26) um eine Gelenkachse (G) relativ zum proximalen Schaftabschnitt (30) abknickbar ist,
wobei die Kopplungselemente (32) in einem Bereich des Scharniergelenks (34) angeordnet sind.

8. Medizinisches Instrument (10) nach Anspruch 7,
wobei ein Abstand (d) zwischen der einen Schwenkachse (S) und der Gelenkachse (G) konstant ist.
